# EUROPÄISCHE PATENTSCHRIFT

(11) **EP 0 368 085 B1**
(45) Veröffentlichungstag und Bekanntmachung des Hinweises auf die Patenterteilung: **08.12.1993**
(21) Anmeldenummer: 89119884.8
(22) Anmeldetag: 26.10.1989
(51) Int. Cl.: C07C 309/26, C07C 303/28, G03F 7/022

(54) **Verfahren zur Herstellung von substituierten 1,2-Naphthochinon-(2)-diazid-4-sulfonsäureestern und deren Verwendung in einem strahlungsempfindlichen Gemisch**
Process for the preparation of substituted 1,2-naphthoquinone-(2)-diazide-4-sulfonate esters, and their use in a light-sensitive mixture
Procédé de préparation d'esters d'acide de 1,2-naphtoquinone-(2)-diazide-4-sulfoniques substitués et leur emploi dans des mélanges photosensibles

(30) Priorität: 04.11.1988 DE 3837499
(43) Veröffentlichungstag der Anmeldung: 16.05.1990
(73) Patentinhaber: HOECHST AKTIENGESELLSCHAFT, 65926 Frankfurt am Main (DE)
(72) Erfinder: Siegel, Herbert, Dr. Dipl.-Chem., D-6238 Hofheim (DE); Scheler, Siegfried, Dr. Dipl.-Chem., D-6200 Wiesbaden (DE)

(56) Entgegenhaltungen:
- EP-A- 0 258 727
- EP-A- 0 283 898
- JOURNAL FÜR PRAKTISCHE CHEMIE, Band 94, 1916, Seiten 24-34, Leipzig, DE; O. FISCHER UND HAMMERSCHMIDT: "2,7-Dioxynaphtalin III. Über den Monomethyläther des 2,7-Dioxynaphtalins"
- JOURNAL OF THE AMERICAN CHEMICAL SOCIETY, Band 80, 1958, Seiten 2257-2263, Columbus, Ohio, US; M.P. CAVA et al.: "Condensed cyclobutane aromatic systems. V. The synthesis of some alpha-diazo-indanones: Ring contraction in the indane series"

## Beschreibung

Die Erfindung betrifft ein Verfahren zur Herstellung von substituierten 1,2-Naphthochinon-(2)-diazid-4-sulfonsäureestern, ausgehend vom entsprechend substituierten β-Naphthol. Die Erfindung betrifft auch ihre Verwendung in einem Strahlungsempfindlichen Gemisch mit dem entsprechende Aufzeichnungsmaterialien hergestellt werden können.

Naphthochinondiazidsulfonsäuren werden als lichtempfindliche Verbindungen für strahlungsempfindliche Gemische, wie zur Herstellung von Photoresistgemischen oder zur Herstellung von Druckformen verwendet (J. Kosar, "Light-Sensitive Systems", John Wiley & Sons, New York, Kap. 7.4, 1965).

Ihre Herstellung geht aus von durch Hydroxylgruppen substituierten Benzol- oder Naphthalinsulfonsäuren, die man mit Natriumnitrit in verdünnter wäßriger Säure nitrosiert und die entstehende Nitrosoverbindung isoliert von nicht umgesetzten Ausgangsstoffen. Anschließend reduziert man die Nitrosoverbindung zur entsprechenden Aminoverbindung, die abgetrennt und erneut in Wasser suspendiert wird, um bei niederigem pH-Wert in Anwesenheit von Natriumnitrit die Diazidverbindung zu bilden.

Nachteilig an diesem Verfahren ist dessen Mehrstufigkeit und die Notwendigkeit, die entstehenden Nebenprodukte in wäßriger Lösung zu halten und vom jeweils als Niederschlag entstandenen Hauptprodukt zu trennen und das Produkt zu reinigen. Hiermit sind neben großen Produktionskosten, niedrige Ausbeute und nicht ausreichende Produktreinheit verbunden.

Aus EP-A-0 283 898 ist ein Verfahren zur Herstellung von durch Halogen, Nitro oder Alkyl substituierten Aryldiazidsulfonsäuren bekannt, bei dem man von durch mindestens eine Hydroxylgruppe substituierten Arylsulfonsäuren ausgeht sowie Nitrosierung und Reduktion der entstandenen Nitrosoverbindung im alkalischen pH-Bereich vornimmt. Anschließend wandelt man die Aminoverbindung in ein Sulfamat um, mischt das Sulfamatderivat mit einem Diazotierungsmittel, und nach Ansäuern erhält man die Aryldiazidsulfonsäure. Bei diesem Verfahren verbleibt das nach jedem Reaktionsschritt entstandene und zur Weiterreaktion geeignete Reaktionsprodukt in Lösung, das Endprodukt ist jedoch nicht frei von isomeren Verbindungen.

Kernsubstituierte 1,2-Naphthochinon-(2)-diazid-4-sulfonsäureester sind für die Herstellung von Photoresistlacken von Interesse. Durch die Substituenten wird ihr Lichtempfindlichkeitsbereich erweitert.

Gemäß der EP-A 0 258 727 werden ortho-Naphthochinondiazidsulfonsäureester hergestellt durch Umsetzen eines ortho-Naphthochinondiazidsulfonsäurehalogenids mit einer ein- oder mehrwertigen phenolischen Verbindung in Gegenwart von Ammoniak, Ammoniumsalzen schwacher Säuren oder von aliphatischen Abkömmlingen des Ammoniaks mit 1 bis 3 Kohlenstoffatomen bei einem pH-Wert im Bereich zwischen etwa 1,5 und 8,5. Die Ester lassen sich in einem lichtempfindlichen Gemisch verwenden.

Für die Produktion derartiger Photolacke ist es wichtig, daß man über ein wirtschaftliches Verfahren zur Herstellung der lichtempfindlichen Komponenten verfügt.

Es war Aufgabe der Erfindung, ein Verfahren zur Herstellung von substituierten 1,2-Naphthochinon-(2)-diazid-4-sulfonsäureestern anzugeben, das es ermöglicht,in einfachen Verfahrensschritten und mit guter Ausbeute die gewünschten Verbindungen herzustellen.

Erfindungsgemäß wird ein Verfahren zur Herstellung von 1,2-Naphthochinon-(2)-diazid-4-sulfonsäureestern der allgemeinen Formel I 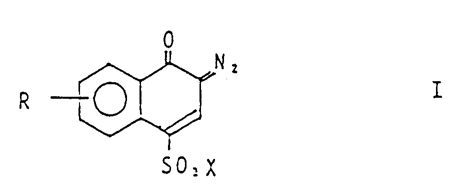
vorgeschlagen, die in mindestens einer der 5-, 6-, 7-oder 8-Position durch R = Halogen, Alkoxy- oder Alkoxy-carbonyl-Gruppen substituiert sind und bei denen X eine Arylgruppe bedeutet, durch die Kombination an sich bekannter Verfahrensschritte, in dem man
a) entsprechend substituiertes β-Naphthol nitrosiert,
b) mit Alkalihydrogensulfit und Säure in 4-Position reduziert und sulfoniert,
c) das Naphthalinsulfonsäurederivat vorzugsweise mit Salpetersäure oxidiert,
d) die gebildete 1,2-Naphthochinon-4-sulfonsäure mit Toluolsulfonsäurehydrazid in organischem Lösungsmittel bei Temperaturen von 20 bis 100 °C umsetzt,
e) die entstandene Naphthochinondiazidverbindung mit Chlorsulfonsäure bzw. Chlorsulfonsäure/Thionylchlorid in das Sulfochlorid umwandelt und dieses
f) abschließend mit einer phenolischen Komponente kondensiert.

Vorzugsweise verwendet man als Ausgangsstoff für das erfindungsgemäße Verfahren β-Naphthole, die durch Brom, Alkoxygruppen mit 1 bis 4 Kohlenstoffatomen, oder Alkoxycarbonylgruppen mit 2 bis 5 Kohlenstoffatomen substituiert sind.

Insbesondere sind β-Naphthole als im Handel erhältliche Ausgangssubstanz geeignet, die durch Brom, Methoxy- oder, im besonderen, durch Methoxycarbonyl-Gruppen substituiert sind. Durch die Erfindung wird erreicht, daß man substituierte 1,2-Naphthochinon-(2)-diazid-4-sulfonsäurederivate in einfacher Weise bei guter Ausbeute erhält. Vorzugsweise entfallen erfindungsgemäß eine Reinigung des jeweiligen Zwischenprodukts durch Umfällen oder Umkristallisation.

Die einzelnen Stufen der erfindungsgemäßen Verfahrenskombination sind an sich bekannt:
1,2-Diazo-Ketone können aus 1,2-Diketonen durch Reaktion mit Toluolsulfonylhydrazid hergestellt werden, wie in J. Am. Chem. Soc. 80, 2257 (1958) beschrieben ist. Es ist weiterhin bekannt, daß diese Reaktion zur Herstellung von ortho-Benzochinondiaziden eingesetzt werden kann. Die in Chem. Ber. 95, 1206 (1962) veröffentlichten Versuche mit unsymmetrisch substituierten o-Benzochinonen zeigen, daß die Reaktion zwar bei den dort beschriebenen Verbindungen mit elektronisch aktivierenden Substituenten eine gewisse Regioselektivität aufweist. Dennoch entstehen in der Regel Isomerengemische, so daß die Methode zur Herstellung einheitlicher unsymmetrischer o-Benzochinondiazide ungeeignet ist. Es war daher auch bei von vorneherein unsymmetrischen 1,2-Naphthochinonderivaten, insbesondere wenn diese sowohl aktivierende als auch desaktivierende Substituenten tragen, mit dem Entstehen von Gemischen zu rechnen. Wegen der daraus resultierenden Ausbeuteverluste war somit nach dieser Methode kein ökonomisches Verfahren zu erwarten.

Überraschenderweise wurde nun gefunden, daß man 1,2-Naphthochinon-(2)-diazid-4-sulfonsäuren der allgemeinen Formel I a 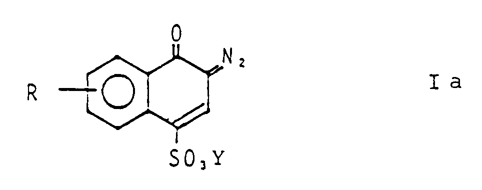
worin
R = Halogen, OR₁ oder -CO-R₁ mit
R₁ = verzweigtes oder unverzweigtes C₁ - C₄ -Alkyl und
Y = H, NH₄ oder Alkalimetall
bedeuten
in hoher Ausbeute und ausgezeichneter Selektivität aus den entsprechenden 1,2-Naphthochinon-4-sulfonsäuren der allgemeinen Formel II erhält, 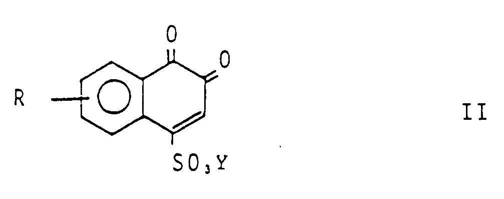
worin R und Y die oben angegebene Bedeutung haben, indem man diese bei einer Temperatur zwischen 20 und 100 °C mit Toluolsulfonsäurehydrazid in einem polaren Lösungsmittel umsetzt. Es ist vor allem überraschend und für eine technische Verwendbarkeit des Verfahrens entscheidend, daß das ebenfalls mögliche, aber unerwünschte Naphthochinondiazid-Isomer der allgemeinen Formel III, 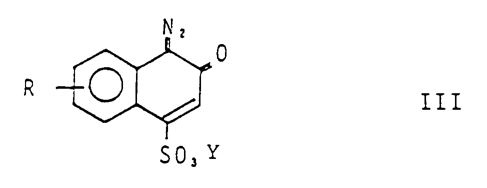
worin R und Y die oben angegebene Bedeutung haben, unabhängig von der Natur des Substituenten R nur in einem unbedeutenden Anteil relativ zum Isomeren der Formel I gebildet wird.

Zur Herstellung der Verbindungen der Formel I geht man so vor, daß man entweder das ortho-Chinon der allg. Formel II in einem polaren organischen Lösungsmittel vorlegt und bei Temperaturen zwischen 0 und 30 °C Toluolsulfonsäurehydrazid zudosiert oder aber das o-Chinon der Formel II zusammen mit dem Toluolsulfonsäurehydrazid als Feststoff vorlegt und bei 0 - 30 °C ein polares organisches Lösungsmittel zugibt. Anschließend erwärmt man das in der Regel als Supension vorliegende Reaktionsgemisch auf Temperaturen zwischen 20 und 100 °C, wobei das Chinon in Lösung geht und das enstandene Chinondiazid ausfällt.

Besonders bevorzugt sind Lösungsmittel wie Alkohole, da in ihnen das gebildete Naphthochinondiazid der Formel I nur schlecht löslich ist und so direkt in hoher Ausbeute aus dem Reaktionsgemisch abgetrennt werden kann.

Das Verfahren ist vor allem deshalb sehr gut zur Herstellung der strahlungsempfindlichen Naphthochinondiazide der allgemeinen Formel I geeignet, da etwa in geringen Mengen enthaltene Isomere der allgemeinen Formel III bei der Weiterverarbeitung zu den Sulfonsäurechloriden der allgemeinen Formel IV 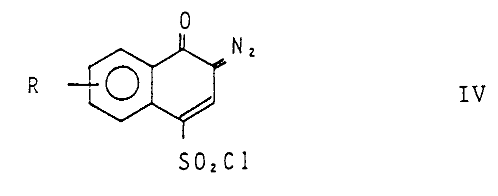
worin R die oben angegebene Bedeutung hat, nicht stören. Nach den zur Herstellung derartiger Verbindungen üblichen Verfahren gewinnt man die Verbindungen der allgemeinen Formel IV z.B. durch Umsetzung mit Chlorsulfonsäure bzw. Chlorsulfonsäure/Thionylchlorid und anschließende Ausfällung mit Wasser. Während Sulfochloride der allgemeinen Formel IV sich leicht isolieren lassen, sind die entsprechenden Sulfochloride der allgemeinen Formel V, 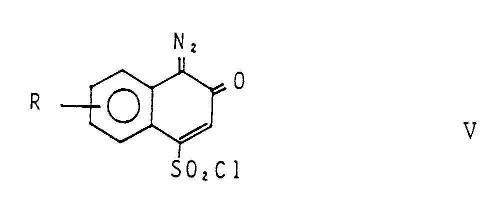
worin R die oben angegebene Bedeutung hat, so hydrolyseempfindlich, daß sie bei der Ausfällung des Reaktionsgemisches sofort zu den wasserlöslichen Sulfonsäuren zersetzt werden.

Durch Kombination der beiden Schritte, nämlich der Umsetzung von 1,2-Naphthochinon-4-sulfonsäuren der allgemeinen Formel I mit Toluolsulfonsäurehydrazid sowie der direkten Umsetzung des Rohprodukts aus dieser Reaktion zum Säurechlorid und anschließender Hydrolyse des Reaktionsgemisches mit Wasser, erhält man direkt isomeren-reines Sulfochlorid der allgemeinen Formel IV.

Das Sulfochlorid der allgemeinen Formel IV ist ein wertvolles Ausgangsmaterial für die Herstellung von lichtempfindlichen Sulfonsäureestern oder kann durch Hydrolyse z.B. mit wäßrigem Alkali wieder in hochreine Sulfonsäure der allgemeinen Formel I umgewandelt werden.

Die Herstellung der Vorstufe der allgemeinen Formel II erfolgt in an sich bekannter Weise über drei Stufen.

In der ersten Stufe nitrosiert man ein β-Naptholderivat der allgemeinen Formel VI, 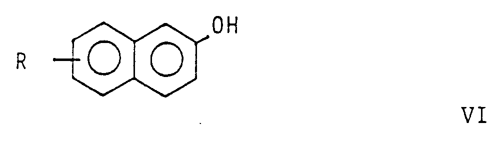
worin R die oben angegebene Bedeutung hat, entsprechend der in J. prakt. Chem. 94, 24 (1916) beschriebenen Methode, indem man es bei einer Temperatur von 0 - 5 °C in Eisessig mit Natriumnitritlösung versetzt und bei Raumtemperatur nachrührt bis alles Ausgangsmaterial umgesetzt ist. Das entstandene ortho-Chinonoxim der allgemeinen Formel VII, 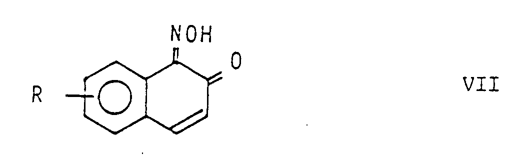
worin R die oben angegebene Bedeutung hat, wird anschließend mit Wasser ausgefällt, isoliert und gegebenenfalls getrocknet. Bei dieser Verfahrensweise fällt das Rohprodukt so rein an, daß es direkt ohne weitere Reinigung, vorzugsweise als säurefrei gewaschenes Feuchtprodukt, weiterverarbeitet werden kann.

In der zweiten Stufe wird das ortho-Chinonoxim der allgemeinen Formel VII zunächst mit Natriumhydrogensulfit in verdünnter Lösung sulfoniert und dann nach Ansäuern mit Schwefelsäure durch freigesetzte schweflige Säure zu der 1-Amino-2-hydroxy-4-naphthalinsulfonsäure der allgemeinen Formel VIII, 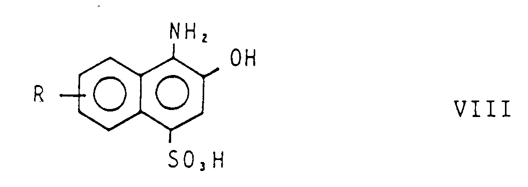
worin R die oben angegebene Bedeutung hat, reduziert. Die Vorgehensweise entspricht teilweise dem in Organic Synthesis, Col. Vol. II, 42 (1943) bei der Herstellung von 1-Amino-2-hydroxy-4-naphthalinsulfonsäure beschriebenen Verfahren. Hier wurde jedoch gefunden, daß es im Falle der ortho-Chinonoxime der allgemeinen Formel VII vorteilhafter ist, die Lösezeit dieser Verbindungen bei der Sulfonierung mit Hydrogensulfit von einigen Minuten auf eine Zeit von bis zu 24 Stunden auszudehnen oder aber beim Lösevorgang die Temperatur auf 30 - 60 °C zu erhöhen. Der Vorteil bei dieser Verfahrensweise ist, daß man weniger Löserückstand erhält und dadurch bessere Ausbeuten erreicht. Dies stellt insbesondere im Falle der schlechter löslichen Verbindungen mit R = Brom eine wesentliche Verbesserung der beschriebenen Methode dar. Man isoliert das Reaktionsprodukt der allgemeinen Formel VIII, indem man es nach dem Ansäuern auskristallieren läßt, die Kristalle abfiltriert und gegebenenfalls trocknet. Die Qualität der erhaltenen Rohprodukte reicht aus, um sie ohne weitere Reinigung - vorzugsweise als wasserfeuchtes Produkt - direkt weiter umzusetzen.

In der dritten Stufe wird die 1-Amino-2-hydroxy-4-naphthalinsulfonsäure der allgemeinen Formel VIII mit einem Oxidationsmittel wie z.B. Salpetersäure zu der bereits weiter oben beschriebenen ortho-1,2-Naphthochinon-4-sulfonsäure der allgemeinen Formel II, 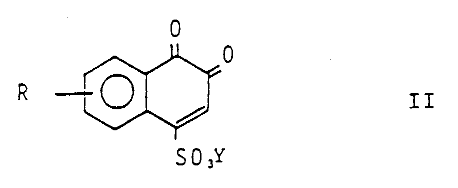
worin R und Y die oben angegebene Bedeutung haben, entsprechend der in Organic Synthesis 21, 91 (1941) angegebenen Methode oxidiert. Man arbeitet zweckmäßiger mit verdünnter Salpetersäure, damit das als Suspension vorliegende Reaktionsgemisch rührfähig ist. Die Oxidation erfolgt bevorzugt bei Raumtemperatur und man isoliert das entstandene Produkt vorzugsweise durch Aussalzen mit Ammoniumchlorid und anschließende Filtration.

Zu dem Zwischenprodukt der allgemeinen Formel II mit R = OR¹, wobei R die oben angegebene Bedeutung hat, gelangt man außerdem in an sich bekannter Weise, indem man nach Chem. Ber. 27,3050 (1894) das o-Chinon der Formel IX herstellt 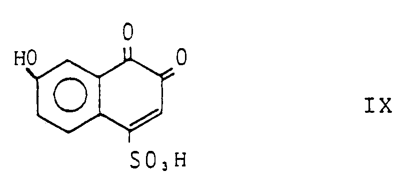
und dieses mit einem Alkylierungsmittel wie Alkylhalogenid, Dialkylsulfat oder Toluolsulfonsäurealkylester umsetzt.

Das so erhaltene Rohprodukt enthält in der Regel geringe Anteile der entsprechenden 1,2-Naphthochinon-1-diazid-4-sulfonsäure der allgemeinen Formel III (s.o.), die sich mit der bei der Oxidation entstehenden salpetrigen Säure unter den Reaktionsbedingungen in einer Nebenreaktion aus dem Ausgangsmaterial der Formel VIII bildet. Diese Verunreinigung stört aber bei der weiteren Umsetzung der rohen ortho-Chinone der Formel II zu den Ortho-Chinondiaziden der Formel I nicht. Wie oben beschrieben, kann sie leicht über die Säurechloridhydrolyse auf der Stufe des Säurechlorids der allgemeinen Formel IV abgetrennt werden. Dies hat den Vorteil, daß auch auf der hier erläuterten Synthesestufe der ortho-Chinone der allgemeinen Formel II keine Reinigungsoperation für das Rohprodukt erforderlich ist und es vorzugsweise nach Trocknung direkt weiterverarbeitet werden kann.

Das Sulfochlorid nach Formel IV ist ein wertvolles Ausgangsprodukt zur Herstellung von erfindungsgemäßen, strahlungsempfindlichen Estern der allgemeinen Formel X 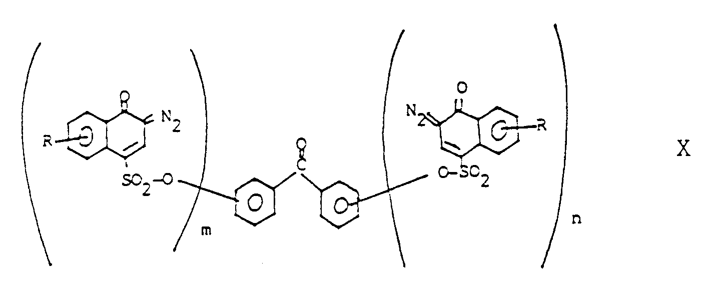
mit m = 1 bis 3 und n = 0 bis 3.

Die Ester der allgemeinen Formel X können durch Überführung von z.B. 6-Brom-1-naphthochino-(2)-diazid-4-sulfonsäure in das entsprechende Säurechlorid sowie dessen Umsetzung mit einem Hydroxy-Benzophenon der allgemeinen Formel XI, 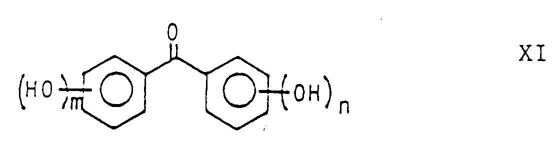
worin m und n die oben angegebene Bedeutung haben, nach dem Stand der Technik entsprechenden Verfahren hergestellt werden. Als besonders vorteilhaft und über den Stand der Technik hinausgehend erweist sich hier jedoch die Verwendung sterisch gehinderter Basen, wie z.B. 1,4-Diazabicyclo[2,2,2]octan (Dabco). Die Ester zeichnen sich durch hohe Lichtempfindlichkeit aus und sind daher geeignet für die Herstellung positiv arbeitender Photolacke.

Als phenolische Komponente können auch andere bekannte Phenole oder deren Derivate wie Novolake dienen.

Die erfindungsgemäß hergestellten Verbindungen, insbesondere die Alkoxycarbonyl-1,2-naphthochinon-(2)-diazid-4-sulfonsäureester, wie der 6-Alkoxycarbonyl-1,2-naphthochinon-(2)-diazid-4-sulfonsäureester, werden als strahlungsempfindliche Komponente in strahlungsempfindlichen Gemischen eingestzt. Die Gemische bestehen im wesentlichen aus der strahlungsempfindlichen Verbindung und einem wasserunlöslichen, in wäßrig-alkalischen Lösungen löslichen oder mindestens quellbaren harzartigen Bindemittel.

In Verbindung mit einem Schichtträger bildet das aufgebrachte, strahlungsempfindliche Gemisch ein strahlungsempfindliches Aufzeichnungsmaterial wie eine Druckplatte oder ein Photoresistmaterial.

Als Bindemittel sind beispielsweise Phenol- und Kresol-Novolake und Polyvinylphenolharze geeignet.

Die alkalischen Novolake und Polyvinylphenolharze, die für die Herstellung von lichtempfindlichen Gemischen verwendet werden können, sind bekannt. Ein Verfahren zur Herstellung solcher Novolake ist in "Chemistry and Application of Phenolic Resins" (Chemie und Anwendung von Phenolharzen), von A. Knop und W. Scheib, Springer Verlag, New York, 1979, Kapitel 4, beschrieben. Die Verwendung von Polyvinylphenolen ist aus US-A 3 869 292 und 4 439 516 bekannt.

Für die Herstellung der erfindungsgemäßen lichtempfindlichen Gemische werden der Novolak oder das Polyvinylphenol und die strahlungsempfindliche Verbindung in einem Lösemittel gelöst. Hierfür geeignete Lösemittel sind beispielsweise Glykolether, wie z.B. Ethylenglykolmonomethylether und Ethylenglykolmonoethylether oder auch deren Acetate wie Propylenglykolmethyletheracetat; Ester, wie z.B. Ethylacetat und Butylacetat; Ketone, wie z.B. Methylethylketon, Cyclopentanon und Cyclohexanon; sowie aromatische Kohlenwasserstoffe, wie z.B. Toluol und Xylol. Gemische aus diesen Lösemitteln können ebenfalls eingesetzt werden.

Dem erfindungsgemäßen strahlungsempfindlichen Gemisch können vor dem Aufbringen auf einen Schichtträger noch Zusätze, wie z.B. Farbmittel, Farbstoffe, Verlaufmittel, Weichmacher, Haftvermittler, Entwicklungsbeschleuniger, Tenside, z.B. nicht-ionische Tenside, und Vernetzungsmittel, zugegeben werden.

In bevorzugter Ausführung liegt der Gehalt an festen Bestandteilen des strahlungsempfindlichen Gemisches für den Novolak oder das Polyvinylphenol bei etwa 15 bis 99 Gewichtsprozent, für die strahlungsempfindliche Verbindung bei etwa 1 bis 85 Gewichtsprozent. Insbesondere ist das Bindemittel in einem Anteil von etwa 50 bis 97 Gewichtsprozent, und ganz besonders bevorzugt von etwa 65 bis 93 Gewichtsprozent, bezogen auf das Gewicht der festen Bestandteile, in dem Gemisch enthalten. Der Anteil an lichtempfindlicher Verbindung liegt insbesondere bei etwa 3 bis 50 Gewichtsprozent und ganz besonders bevorzugt bei etwa 7 bis 35 Gewichtsprozent, bezogen auf das Gewicht der festen Bestandteile des Gemisches.

Erfindungsgemäß wird ferner ein strahlungsempfindliches Aufzeichnungsmaterial vorgeschlagen, bestehend aus dem oben beschriebenen lichtempfindlichen Gemisch und einem Schichtträger.

Das strahlungsempfindliche Gemisch kann nach einem der üblichen Verfahren, wie Tauchen, Sprühen und Aufschleudern, auf den Schichtträger gebracht werden. Beim Aufschleudern kann zum Beispiel der Prozentanteil an Feststoffen in der Resistlösung so eingestellt werden, daß sich in Abhängigkeit von der im Einzelfall verwendeten Aufschleudervorrichtung und der für den Aufschleudervorgang angesetzten Zeitspanne eine Beschichtung in der gewünschten Dicke ergibt. Beispiele für geeignete Trägermaterialien sind: Silicium, Aluminium oder polymere Harze, Siliciumdioxid, dotiertes Siliciumdioxid, Siliciumnitrid, Tantal, Kupfer, polykristallines Silicium (polysilicon), Keramik und Aluminium/Kupfer-Legierungen.

Die Erfindung wird durch die folgenden Beispiele naher erläutert, ohne sie hierauf zu beschränken:

### Beispiel 1:

### 7-Methoxy-1,2-naphthochinon-(2)-diazid-4-sulfonsäure-Ammoniumsalz

35,6 g (0,125 Mol) 7-Methoxy-1,2-naphthochinonsulfonsäure-Ammoniumsalz und 23,5 g (0,126 Mol) Toluolsulfonsäurehydrazid wurden in 285 ml Methanol aufgeschlämmt und die Suspension eine Stunde auf 45 °C erwärmt Danach wurde das Reaktionsgemisch auf 2 °C abgekühlt und die Kristalle abgesaugt. Man erhielt 26,4 g (71 %) 7-Methoxy-1-naphthochinon-(2)-diazid-4-sulfonsäure als Ammoniumsalz. Zersetzungspunkt FP: 151 °C
NMR (DMSO): 3,862 ppm (s; 3 H; CH₃); 7,301 ppm (d d; J₁ = 9,1 Hz; J₂ = 2,9 Hz; H an C-6); 7,567 ppm (d; J = 2,9 Hz; H an C-8); 7,677 ppm (s; H an C-3); 8,461 ppm (d; J = 9, 1 Hz; H an C-5); 7,920 (t; J = 51,3 Hz; NH₄⁺).

Das Produkt kann bei Verwendung von rohem ortho-Chinon noch geringe Mengen des isomeren 7-Methoxy-1,2-naphthochinon-(1)-diazid-4-sulfonsäure-Ammoniumsalzes als Verunreinigung, die aus der Vorstufe stammt, enthalten. Bei der Umsetzung zum Säurechlorid analog Beispiel 3 wird dieses Isomere jedoch infolge Hydrolyse vollständig entfernt, so daß auf der Stufe des hier anfallenden Ammoniumsalzes keine weitere Reinigung erforderlich ist.

### Herstellung der Vorstufen:

### a) 1-Nitroso-7-methoxy-β-naphthol

110,2 g (0,633 Mol) 7-Methoxy-β-naphthol wurden in einem Gemisch aus 650 ml Eisessig und 65 ml Wasser bei 0 °C mit einer Lösung von 45,0 g (0,65 mol) Natriumnitrit in 340 ml Wasser innerhalb von zwei Stunden nitrosiert. Man rührte noch zwei Stunden nach und ließ die Temperatur des Reaktionsgemisches in dieser Zeit auf 22 °C ansteigen. Anschließend füllte man mit Wasser auf ein Volumen von 1,6 l auf und saugte das ausgefallene Produkt ab. Nach Trocknung erhielt man 120,3 g (93,5 %) 1-Nitroso-7-methoxy-β-naphthol.
FP: 122 - 124 °C
NMR (DMSO): 3,855 ppm (s; CH₃); 6,276 ppm (d; J = 9,8 Hz; 1 H); 7,079 ppm (dd; J₁ = 2,6 Hz; J₂ = 8,5 Hz; 1 H); 7,515 ppm (d; J = 8,5 Hz; 1 H); 7,650 ppm (d; J = 9,8 Hz; 1 H); 8,222 ppm (breites s, 1 H).

### b) 1-Amino-2-hydroxy-7-methoxynaphthalin-4-sulfonsäure

168,4 g (1,62 Mol) Natriumhydrogensulfit wurden in 350 ml Wasser gelöst. Zu dieser Lösung gab man 28 ml 6-normale Natronlauge. Anschließend wurde auf 10 °C abgekühlt und 120,1 g (0,59 Mol) rohes 1-Nitroso-7-methoxy-β-naphthol eingerührt. Man verdünnte dann mit Wasser auf ein Volumen von 760 ml, wobei sich eine Temperatur von 23 °C einstellte. Man rührte 16 Stunden bei Raumtemperatur weiter und filtrierte dann 9,5 g unlöslichen Rückstand ab. Anschließend füllte man das Filtrat mit Wasser auf 1250 ml auf und gab 206,8 g (2,03 Mol) 96 %ige Schwefelsäure zu, wobei die Temperatur um 10 °C anstieg. Man ließ zwei Tage bei Raumtemperatur stehen und saugte dann die ausgefallenen Kristalle ab. Man erhielt nach Trocknung 108,5 g (68,4 %) 1-Amino-2-hydroxy-7-methoxynaphthalin-4-sulfonsäure.
FP: 275 °C (Zersetz.)
NMR (DMSO): 3,886 ppm (s; CH₃); 4,029 ppm (breites s, 4 H) ; 7,049 ppm (dd; J₁ = 2,3 Hz; J₂ = 9,3 Hz; 1 H); 7,200 ppm (d; J = 2,3 Hz; 1 H); 7,653 ppm (s; 1 H); 8,696 ppm (d; J = 9,3 Hz; 1 H)

### c) 7-Methoxy-1,2-naphthochinon-4-sulfonsäure Ammoniumsalz

29,6 ml (0,556 Mol) 70,4 %ige Salpetersäure wurden mit 82 ml Wasser verdünnt. Zu dieser Lösung des Oxidationsmittels gab man bei maximal 30 °C innerhalb einer Stunde unter Rühren 80,0 g (0,298 Mol) rohe 1-Amino-2-hydroxy-7-methoxynaphthalin-4-sulfonsäure. Man rührte 1 Stunde nach, kühlte auf 10 °C ab und gab eine Lösung von 17,6 g Ammoniumchlorid in 46 ml Wasser zu. Die Suspension wurde zwei Stunden auf 0°C abgekühlt und das Produkt abgesaugt. Die Kristalle wurden zuerst mit 25 ml gesättigter Ammoniumchloridlösung und dann mit 40 ml Ethanol nachgewaschen. Man erhielt nach Trocknung 77,4 g (91,3 %) rohes 7-Methoxy-1,2-naphthochinon-4-sulfonsäure-Ammoniumsalz.
FP: 210 °C (Zersetz.)
NMR (DMSO): 3,859 ppm (s, CH₃); 6,559 ppm (s, H an C-3); 7,274 ppm (dd; J₁ = 8,7 Hz; J₂ = 2,9 Hz; H an C-6); 7,411 ppm (d; J = 2,9 Hz; H an C-8); 8,320 (d; J = 8,7 Hz; H an C-5); 7,560 (t; J = 51,1 Hz; NH₄⁺)

Das Produkt enthält bei dieser Verfahrensweise geringe Mengen an 7-Methoxy-1,2-naphthochinon-1-diazid-4-sulfonsäure-Ammoniumsalz.
NMR (DMSO): 3,868 ppm (s, CH₃); 6,834 ppm (s, H an C-3); 6,894 ppm (dd; J₁ = 2,5 Hz; J₂ = 9,1 Hz; H an C-6); 7,020 ppm (d; J = 2,5 Hz; H an C-8); 8,494 (d; J = 9,1 Hz; H an C-5); 7,560 (t; J = 51,1 Hz; NH₄⁺)

Die Reinheit des Rohprodukts ist jedoch für die weiteren Reaktionen ausreichend.

### Beispiel 2:

### 6-Brom-1,2-naphthochino-(2)-diazid-4-sulfonsäure-Ammoniumsalz

71,0 g (0,213 Mol) 6-Brom-1,2-naphthochinon-4-sulfonsäure-Ammoniumsalz und 40,2 g (0,216 Mol) Toluolsulfonsäurehydrazid wurden in 475 ml Methanol aufgeschlämmt und die Suspension zwei Stunden auf 44 °C erwärmt. Danch wurde das Reaktionsgemisch auf 2 °C abgekühlt und die Kristalle abgesaugt.

Man erhielt 62,1 g (84 %) 6-Brom-1,2-naphthochinon-(2)-diazid-4-sulfonsäure-Ammoniumsalz. Aus der Mutterlauge konnten durch Einengen und Digerieren mit Ethylacetat weitere 11,7 g (16 %) Produkt gewonnen werden, so daß die Gesamtausbeute an Chinondiazid praktisch quantitativ ist.
Zersetzungspunkt: 235 °C
NMR (DMSO): 7,638 ppm (dd; J₁ = 8,54 Hz; J₂ = 1,99 Hz; H an C-7); 7,905 ppm (s; H an C-3); 8,053 ppm (d; J = 8,54 Hz; H an C-8); 8,691 (d; J = 1,99 Hz; an C-5); 7,65 ppm (t; J = 51,2 Hz; NH₄⁺)

Das hier erhaltene Ammoniumsalz kann bei Verwendung von rohem o-Chinon noch geringe Mengen des isomeren Chinondiazids enthalten. Bei der Umsetzung zum Säurechlorid wird das Isomere jedoch infolge Hydrolyse vollständig entfernt, so daß auf der Stufe des hier anfallenden Ammoniumsalzes keine weitere Reinigung erforderlich ist.

### Herstellung der Vorstufen:

### a) 1-Nitroso-6-brom-β-naphthol

76,2 g (0,342 Mol) 6-Brom-β-naphthol wurden in 385 ml 90 %iger Essigsäure aufgeschlämmt und bei 4 °C mit 28,5 g (0,413 Mol) Natriumnitrit in 230 ml Wasser während zwei Stunden nitrosiert und anschließend 23 h bei Raumtemperatur nachgerührt. Die Suspension wurde dann mit Wasser auf das doppelte Volumen aufgefüllt und das entstandene Produkt abgesaugt. Man erhielt nach dem Trocknen 83,8 g (97,3 %) rohes 1-Nitroso-6-brom-β-naphthol, dessen Reinheit für die weitere Umsetzung ausreichend ist.
FP: 144 - 148 °C
NMR (DMSO): 6,48 (d; J = 10,3 Hz; 1 H); 7,65 (s; 1 H); 7,75 (d; J = 2,1 Hz; 1 H) 7,68 (d; J = 2,1 Hz; 1 H); 8,65 (d; J = 10,3 Hz, 1 H)

### b) 1-Amino-2-hydroxy-6-brom-4-naphthalinsulfonsäure

84,8 g (0,337 Mol) rohes 1-Nitroso-6-brom-β-naphthol wurden bei Raumtemperatur in eine Lösung von 97,5 g (0,939 Mol) Natriumhydrogensulfit und 4,0 g (0,1 Mol) Natronlauge in 376 ml Wasser eingerührt. Die Suspension wurde mit Wasser auf 1350 ml aufgefüllt und 24 Stunden bei Raumtemperatur gerührt. Danach wurden 6,8 g (8 % der Einwaage) Löserückstand durch Filtration abgetrennt. Anschließend wurde bei maximal 30 °C mit 119,4 g (1,17 Mol) 96 %iger Schwefelsäure angesäuert und das Produkt 48 Stunden bei Raumtemperatur auskristallisiert. Nach Absaugen und Trocknen erhielt man 92,0 g (86 %) 1-Amino-2-hydroxy-6-brom-4-naphthalinsulfonsäure.
FP: 270 °C (Zersetz.)
NMR (DMSO): 7,73 ppm (d; J = 1,5 Hz; 1 H); 7,77 ppm (s; 1 H); 7,86 ppm (s; 1 H); 8,95 ppm (d; J = 1 ,5 Hz, 1 H); 5,42 ppm (breites s; 4 H)

### c) 6-Brom-1,2-naphthochinon-4-sulfonsäure-Ammoniumsalz

80 g (0,252 Mol) rohe 1-Amino-2-hydroxy-6-brom-4-naphthalinsulfonsäure wurden unter Rühren portionsweise bei 22 - 27 °C innerhalb von 80 Minuten in 105 ml 24 %ige (0,478 Mol) Salpetersäure eingetragen. Es entstand eine dickflüßige Suspension, die anschließend auf 6 °C abgekühlt und mit 14,7 g (0,275 Mol) Ammoniumchlorid in 38 ml Wasser versetzt wurde. Man rührte noch 30 Minuten bei 3 °C weiter und saugte das entstandene ortho-Chinon ab. Die Kristalle wurden nacheinander mit 40 ml gesättigter Ammoniumchloridlösung und 20 ml Ethanol gewaschen. Nach Trocknung erhielt man 71,4 g (85 %) rohe 6-Brom-1,2-naphthochinon-4-sulfonsäure als Ammoniumsalz.
FP: 225 °C (Zersetz.)
NMR (DMSO): 6,78 ppm (s; 1 H); 7,83 ppm (d; J = 1,7 Hz, 1 H); 7,86 ppm (s; 1 H); 8,56 ppm (d; J = 1,7 Hz; 1 H); 7,13 ppm (t; J = 52,8 Hz; NH₄⁺)

Das Produkt erhält bei dieser Verfahrensweise geringe Mengen 6-Brom-1,2-naphthochinon-1-diazid-4-sulfonsäure-Ammoniumsalz.
NMR (DMSO): 7,008 ppm (s; H an C-3); 7,525 ppm (d; J = 8,59 Hz; H an C-8); 7,684 ppm (dd; J₁ = 8,59 Hz; J₂ = 2,13 Hz; H an C-7); 8,728 ppm (d; J = 2,13 Hz; H an C-5)

### Beispiel 3:

### 6-Brom-1,2-naphthochino-(2)-diazid-4-sulfonsäurechlorid

60,0 g (0,174 Mol) 6-Brom-1,2-naphthochinon-(2)-diazid-4-sulfonsäure-Ammoniumsalz wurden innerhalb von 40 Minuten in ein Gemisch aus 154 ml (2,31 Mol) Chlorsulfonsäure und 48 ml (0,66 Mol) Thionylchlorid eingetragen. Die Temperatur stieg dabei von 19 auf 40 °C an. Man erwärmte noch 45 Minuten auf 60 °C und kühlte dann auf Raumtemperatur ab. Das Reaktionsgemisch wurde anschließend auf Eiswasser gegeben, wobei das entstandene Säurechlorid ausfiel. Das Produkt wurde auf einer Nutsche isoliert und getrocknet. Man erhielt 58,0 g (96 %,) isomeren-reines 6-Brom-1,2-naphthochinon-(2)-diazid-4-sulfonsäurechlorid als gelben Feststoff.
FP: 182 °C (Zersetz.)
NMR (DMSO): 7,62 ppm (d; J = 8,9 Hz; 1 H); 8,06 (s; H an C-3); 8,11 (d; J = 8,9 Hz; 1 H); 8,73 (s; H an C-5)

### Beispiel 4:

### 2,3,4-Tris-(6-brom-1,2-naphthochinon-(2)-diazid-4-sulfonyloxy)-benzophenon

28,0 g (0,081 Mol) 6-Brom-1,2-naphthochinon-(2)-diazid-4-sulfonsäurechlorid und 6,2 g (0,027 Mol) 2,3,4-Trihydroxybenzophenon wurden in 94 ml Acetonitril vorgelegt. Innerhalb von 30 Minuten gab man 8,8 g (0,087 Mol) N-Methylmorpholin zu, wobei das Ausgangsmaterial in Lösung ging und die Temperatur von 17 °C auf 30 °C anstieg. Man rührte noch 1,5 Stunden bei Raumtemperatur nach und säuerte dann mit 3,2 ml Eisessig an. Das Reaktionsgemisch wurde anschließend in 1 l Wasser eingerührt. Das ausgefallene Produkt wurde abgesaugt, mit Wasser neutral gewaschen und bei 35 °C getrocknet. Man erhielt 30,6 g (97,5 %) Trisester als gelbes Pulver.
FP: 155 °C (Zersetz.)
N_{gef.} : 6,8 % N_{ber.}: 7,2 %
H₂O : 1,0 %

### Beispiel 5:

### Gemisch aus Estern von 6-Brom-1,2-naphthochino-(2)-diazid-4-sulfonsäure und 1,2-Naphthochinon-(2)-diazid-5-sulfonsäure und 2,3,4-Trihydroxybenzophenon

25,7 g (0,074 Mol) 6-Brom-1,2-naphthochinon-(2)-diazid-4-sulfonsäurechlorid, 2,2 g (0,0082 Mol) 1,2-Naphthochinon-(2)-diazid-5-sulfonsäurechlorid und 6,3 g (0,027 Mol) 2,3,4-Trihydroxybenzophenon wurden in 96 ml Acetonitril vorgelegt. Innerhalb von 20 Minuten gab man 9,0 g (0,089 Mol) N-Methylmorpholin zu, wobei die Temperatur von 17 °C auf 33 °C anstieg und das Ausgangsmaterial in Lösung ging. Man rührte noch 1,5 Stunden nach und säuerte dann mit 7 ml 30 %iger Salzsäure an. Das Reaktionsgemisch wurde anschließend in 1 l Wasser eingerührt. Das ausgefallene Produkt wurde abgesaugt und bei 35 °C getrocknet. Man erhielt 30,3 g (98,5 %) Estergemisch als gelbes Pulver.
FP: 155 °C (Zersetz.)
N_{gef.}: 7,4 % N_{ber.}: 7,4 %
H₂O : 1,2 %

### Beispiel 6:

### 2,3,4-Tris-(6-methoxycarbonyl-1,2-naphthochinon-(2)-diazid-4-sulfonyl-oxy)-benzophenon

### Herstellung der Vorstufen:

### a) 1-Nitroso-6-methoxycarbonyl-β-naphthol

202,0 g (1,0 Mol) 6-Methoxycarbonyl-β-naphthol wurden in 1120 ml 90 %iger Essigsäure aufgeschlämmt und bei 0 - 5 °C innerhalb von 2,5 Stunden mit 182 g (1,05 Mol) 40 %iger Natriumnitritlösung nitrosiert und anschließend 45 Minuten bei Raumtemperatur nachgerührt. Die erhaltene Suspension wurde mit Wasser auf 3 l aufgefüllt, der Feststoff abgesaugt und mit Wasser neutral gewaschen. Man erhielt nach dem Trocknen 228,1 g (99,7 %) rohes 1-Nitroso-6-methoxycarbonyl-β-naphthol.
FP: 175 °C (Zersetz.)
NMR (DMSO): 3,90 ppm (s; CH₃); 6,50 ppm ,(d; J = 9,9 Hz; 1 H); 7,87 ppm (d; J = 9,9 Hz; 1 H); 8,04 ppm (dd; J₁ = 8,4 Hz; J₂ = 1,8 Hz; 1 H); 8,16 ppm (d; J = 1,8 Hz; 1 H); 8,82 ppm (d; J = 8,4 Hz; 1 H)

### b) 1-Amino-2-hydroxy-4-sulfo-6-naphthalincarbonsäuremethylester

92,4 g (0,4 Mol) rohes 1-Nitroso-6-methoxycarbonyl-β-naphthol wurden bei Raumtemperatur zu einer Lösung aus 360 g (1,11 Mol) 40 %igem wäßrigem Natriumhydrogensulfit und 4,4 g (0,11 Mol) Natriumhydroxid in 1610 ml Wasser gegeben. Man erwärmte die Suspension 24 h auf 40 °C und trennte dann 2,4 g (2,6 % der Einwaage) Rückstand ab. Die klare Lösung wurde anschließend bei 25 °C mit 142 g (1,39 Mol) 36 %iger Schwefelsäure angesäuert und das Produkt 48 Stunden bei Raumtemperatur auskristallisiert. Nach Absaugen und Trocknen erhält man 76,0 g (64 %) Produkt.
FP: 280 °C (Zersetz.)
NMR (DMSO): 3,91 ppm (s; CH₃); 7,91 ppm (s; 1 H); 7,90-8,00 (m; 2 H); 9,52 ppm (s; 1 H)

### c) 6-Methoxycarbonyl-1,2-naphthochinon-4-sulfonsäure-Ammoniumsalz

90 g (0,3 Mol) roher 1-Amino-2-hydroxy-4-sulfo-6-naphthalincarbonsäuremethylester wurden bei 25 - 30 °C innerhalb von 90 Minuten in 116 ml 24 %iger (0,57 Mol) Salpetersäure eingetragen. Es entstand eine dickflüssige Suspension, die auf 5 °C abgekühlt und mit 17,5 g (0,325 Mol) Ammoniumchlorid in 45 ml Wasser versetzt wurde. Man rührte noch ein Stunde bei 5 °C weiter und saugte das entstandene ortho-Chinon ab. Die Kristalle wurden mit 45 ml Ethanol gewaschen. Nach der Trocknung erhielt man 75,0 g (79 %) rohe 6-Methoxycarbonyl-1,2-naphthochinon-4-sulfonsäure als Ammoniumsalz.
FP: 212 °C (Zersetz.)
NMR (DMSO): 3,92 ppm (s; CH₃); 6,80 ppm (s; 1 H); 8,07-8,1 ppm (m; 2 H); 9,02 ppm (s; 1 H); 7,13 ppm (t; J = 51,2 Hz; NH₄⁺)

Das Produkt kann bei dieser Verfahrensweise noch geringe Mengen an 6-Methoxycarbonyl-1,2-naphthochinon-(1)-diazid-4-sulfonsäure-Ammoniumsalz enthalten.
NMR (DMSO): 3,89 ppm (s; CH₃); 7,10 ppm (s; H an C-3); 7,65 ppm (d; J = 8,5 Hz; H an C-8); 8,03 ppm (dd; J₁ = 8,5 Hz; J₂ = 1,8 Hz; H an C-7); 9,24 ppm (d; J = 1,8 Hz; H an C-5)

### d) 6-Methoxycarbonyl-1,2-naphthochinon-(2)-diazid-4-sulfonsäure-Ammoniumsalz

69,8 g (0,223 Mol) 6-Methoxycarbonyl-1,2-naphthochinon-4-sulfonsäure-Ammoniumsalz und 42,3 g (0,227 Mol) Toluolsulfonsäurehydrazid wurden in 450 ml Methanol aufgeschlämmt und die Suspension eine Stunde auf 40 °C erwärmt. Man kühlte dann auf 2 °C ab und isolierte das Produkt auf einer Nutsche. Nach Trocknung erhielt man 61,7 g (85,1 %) 6-Methoxycarbonyl-1,2-naphthochinon-2-diazid-4-sulfonsäure-Ammoniumsalz.
FP: 145 °C (Zersetz.)
NMR (DMSO): 3,93 ppm (s; CH₃); 7,97 ppm (s; H an C-3); 8,00 ppm (dd; J₁ = 8,3 Hz; J₂ = 1,7 Hz; H an C-7); 8,28 ppm (d; J = 8,3 Hz; H an C-8); 9,20 ppm (d; J = 1,7 Hz; H an C-5); 7,12 ppm (t; J = 51,0 Hz; NH₄⁺)

Eventuell enthaltene geringe Mengen des isomeren Naphthochinondiazids werden auf der Folgestufe durch Säurechloridhydrolyse vollständig entfernt. Eine Reinigung der hier erhaltenen Rohprodukte entfällt daher.

### e) 6-Methoxycarbonyl-1,2-naphthochinon-(2)-diazid-4-sulfonsäurechlorid

60,0 g (0,185 Mol) 6-Methoxycarbonyl-1,2-naphthochinon-(2)-diazid-4-sulfonsäure-Ammoniumsalz wurden innerhalb von 30 Minuten in ein Gemisch aus 172 ml (2,58 Mol) Chlorsulfonsäure und 54 ml (0,738 Mol) Thionylchlorid eingebracht. Die Temperatur des Gemisches stieg dabei von 24 auf 38 °C an. Man erwärmte noch 1 Stunde auf 50 °C, kühlte auf Raumtemperatur ab und hydrolisierte mit Eiswasser, wobei das enstandene Säurechlorid ausfiel. Nach Isolierung und Trocknung erhielt man 52,7 g (87,2 %) isomerenreines 6-Methoxycarbonyl-1,2-naphthochinon-(2)-diazid-4-sulfonsäurechlorid als gelben Feststoff.
FP: 180 °C (Zersetz.)
NMR (DMSO): 3,93 ppm (s; CH₃); 7,95 ppm (s; H an C-3); 7,98 ppm (dd; J₁ = 8,4 Hz; J₂ = 1,7 Hz; H an C-7); 8,27 ppm (d; J = 8,4 Hz; H an C-8); 9,19 ppm (d; J = 1,7 Hz; H an C-5)

### f) 2,3,4-Tris-(6-methoxycarbonyl-1,2-naphthochinon-(2)-diazid-4-sulfonyloxy)-benzophenon

15,0 g (0,046 Mol) 6-Methoxycarbonyl-1,2-naphthochinon-(2)-diazid-4-sulfonsäurechlorid und 3,52 g (0,0153 Mol) 2,3,4-Trihydroxybenzophenon wurden in 240 ml Acetonitril vorgelegt. Unter Stickstoff wurden bei 25 °C 2,58 g (0,024 Mol) 1,4-Diaza[2,2,2]bicyclooctan (Dabco) zugegeben. Die Temperatur stieg dabei auf 29 °C an, und das Ausgangsmaterial ging in Lösung. Nach 30 Minuten gab man nochmals 0,5 g (0,0044 Mol) Dabco zu und ließ weitere 3 Stunden ausreagieren. Das Reaktionsgemisch wurde mit 10 ml 30 %iger Salzsäure angesäuert und anschließend in 720 ml Wasser eingerührt. Das ausgefallene Produkt wurde abgesaugt, mit Wasser neutralgewaschen und bei 30 °C getrocknet. Man erhielt 14,9 g (88,4 %) Trisester als gelbes Pulver.
FP: 200 °C (Zersetz.)
N_{gef.}: 7,11 % N_{ber.}: 7,64 %
H₂O : 1,6 %

### Beispiel 7:

### 6-Methoxycarbonyl-1,2-naphthochinon-(2)-diazid-4-sulfonsäure-p-cumylphenolester

11,3 g (0,0346 Mol) 6-Methoxycarbonyl-1,2-naphthochinon-(2)-diazid-4-sulfonsäurechlorid und 7,34 g (0,0346 Mol) p-Cumylphenol wurden in 350 ml Acetonitril vorgelegt. Bei 25 °C wurden unter Stickstoff innerhalb von 10 Minuten 4,54 g (0,045 Mol) N-Methylmorpholin zugegeben, wobei das Ausgangsmaterial in Lösung ging. Nach 3 Stunden wurden nochmals 0,92 g (0,009 Mol) N-Methylmorpholin zugegeben, 30 Minuten weitergerührt und dann mit 6 ml 30 %iger Salzsäure versetzt. Anschließend gab man innerhalb von 45 Minuten 1,035 l Wasser zu dem Reaktionsgemisch, saugte das ausgefallene Produkt ab, wusch mit Wasser neutral und trocknete bei 30 °C. Man erhielt 14,0 g (80,6 %) p-Cumylphenolester als gelbe Kristalle.
FP: 156 °C (Zersetz.)
N_{gef.}: 5,8 % N_{ber.}: 5,6 %
H₂O : 0,3 %

### Beispiel 8:

Mit einer Lösung aus
70.00 Gewichtsteile (Gt) Cyclohexanon
3.00 Gt 6-Methoxycarbonyl-1,2-naphthochinon-(2)-diazid-4-sulfonsäure-p-cumylphenolester
27.00 Gt Kresol-Formaldehyd-Novolak (Schmelzbereich 122 - 132 °C nach DIN 53181)
werden Siliciumwafer auf einer Lackschleuder bei einer Drehzahl von 5000 U/min beschichtet und anschließend auf einer Heizplatte bei einer Temperatur von 110 °C 60 s lang getrocknet. Die Schichtdicke beträgt ca. 2,1 µm. Mit einem Projektionsbelichtungsgerät vom Typ FPA 1550 der Firma Canon wird bei einer Wellenlänge von 436 nm durch eine Photomaske, die verschiedene Strichgitter im Größenbereich von 2,0 bis 0,65 µm enthält, belichtet. Die belichteten Wafer werden in einem Tauchverfahren mit einem Entwickler der Marke "AZ-Developer 30" (Hersteller Hoechst AG) 180 s lang entwickelt, anschließend mit Wasser gespült und getrocknet. Man erhält ein positives Abbild der Photomaske, wobei bei einer Belichtungsenergie von 680 mJ/cm² noch Linien und Gräben der Breite 0,9 µm einwandfrei aufgelöst sind.

## Patentansprüche

1. Verfahren zur Herstellung von 1,2-Naphthochinon-(2)-diazid-4-sulfonsäureestern der allgemeinen Formel I 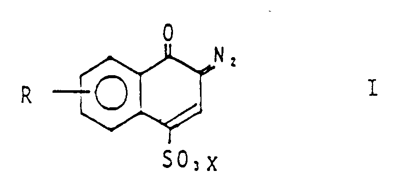 die in mindestens einer der 5-, 6-, 7- oder 8-Position durch R = Halogen, Alkoxy- oder Alkoxycarbonyl-Gruppen substituiert sind, und bei denen X eine Arylgruppe bedeutet, durch die Kombination an sich bekannter Verfahrensschritte, in dem man
a) entsprechend substituiertes β-Naphthol nitrosiert,
b) mit Alkalihydrogensulfit und Säure in 4-Position sulfoniert und reduziert,
c) das Naphthalinsulfonsäurederivat oxidiert,
d) die gebildete 1,2-Naphthochinon-4-sulfonsäure mit Toluolsulfonsäurehydrazid in organischem Lösungsmittel bei Temperaturen von 20 bis 100 °C umsetzt,
e) die Naphthochinon-diazidverbindung mit Chlorsulfonsäure bzw. Chlorsulfonsäure/Thionylchlorid in das Sulfochlorid umwandelt und dieses
f) abschließend mit einer phenolischen Komponente kondensiert.

2. Verfahren nach Anspruch 1, dadurch gekennzeichnet, daß man β-Naphthole verwendet, die durch Brom, Alkoxygruppen mit 1 bis 4 Kohlenstoffatomen, oder Alkoxycarbonylgruppen mit 2 bis 5 Kohlenstoffatomen substituiert sind.

3. Verfahren nach Anspruch 1 oder 2, dadurch gekennzeichnet, daß man β-Naphthole verwendet, die durch Methoxy- oder Methoxycarbonyl-Gruppen substituiert sind.

4. Verfahren nach Anspruch 3, dadurch gekennzeichnet, daß man ein β-Naphthol verwendet, das durch Methoxycarbonyl-Gruppen substituiert ist.

5. Verfahren nach Ansprüchen 1 bis 4, dadurch gekennzeichnet, daß man zur Sulfochloridherstellung das Gemisch der Isomeren 1,2-Naphthochinon-(2)-diazid-4-sulfonsäure und 1,2-Naphthochinon-(1)-diazid-4-sulfonsäure verwendet und die Trennung der Isomeren durch Hydrolyse durchführt.

6. Verfahren nach Ansprüchen 1 bis 5, dadurch gekennzeichnet, daß man zur Kondensation mit Phenolen sterisch gehinderte Basen, vorzugsweise 1,4-Diazabicyclo[2,2,2]octan verwendet.

7. Verfahren nach Ansprüchen 1 bis 6, dadurch gekennzeichnet, daß man als phenolische Komponente ein Hydroxybenzophenonderivat oder einen Novolak einsetzt.

8. Verwendung des 6-Alkoxycarbonyl-1,2-naphthochinon-(2)-diazid-4-sulfonsäureesters nach Ansprüchen 1 bis 4 als strahlungsempfindliche Komponente in einem strahlungsempfindlichen Gemisch.

## Claims

1. A process for preparing 1,2-naphthoquinone-(2)-diazide-4-sulfonic acid esters of the general formula I 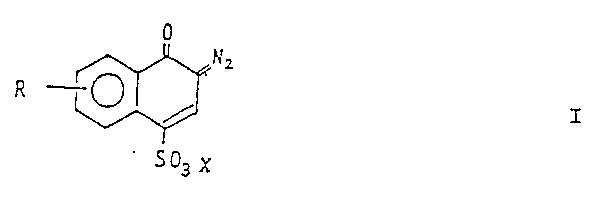 which are substituted in at least one of the positions 5, 6, 7 or 8 by R = halogen, alkoxy groups or alkoxycarbonyl groups, and in which X denotes an aryl group, through combining process steps known per se in which
a) suitably substituted β-naphthol is nitrosated,
b) sulfonation with alkali hydrogensulfite and acid in position 4 and reduction are carried out,
c) the naphthalenesulfonic acid derivative is oxidized,
d) the 1,2-naphthoquinone-4-sulfonic acid formed is reacted with toluenesulfonohydrazide in an organic solvent at temperatures from 20 to 100°C,
e) the naphthoquinonediazide compound is converted with chlorosulfonic acid or chlorosulfonic acid/thionyl chloride into the sulfonyl chloride, and the latter
f) is condensed, in conclusion, with a phenolic component.

2. The process as claimed in claim 1, wherein β-naphthols which are substituted by bromine, alkoxy groups containing 1 to 4 carbon atoms, or alkoxycarbonyl groups containing 2 to 5 carbon atoms, are used.

3. The process as claimed in claim 1 or 2, wherein β-naphthols which are substituted by methoxy groups or methoxycarbonyl groups are used.

4. The process as claimed in claim 3, wherein β-naphthols which are substituted by methoxycarbonyl groups are used.

5. The process as claimed in claims 1 to 4, wherein the mixture of the isomers 1_{,}2-naphthoquinone-(2)-diazide-4-sulfonic acid and 1_{,}2-naphthoquinone-(1)-diazide-4-sulfonic acid is used to prepare the sulfonyl chloride and the isomers are separated by hydrolysis.

6. The process as claimed in claims 1 to 5, wherein sterically hindered bases, preferably 1,4-diazabicyclo[2.2.2]octane, are used for condensation with phenols.

7. The process as claimed in claims 1 to 6, wherein a hydroxybenzophenone derivative or a novolak is used as the phenolic component.

8. The use of the 6-alkoxycarbonyl-1,2-naphthoquinone-(2)-diazide-4-sulfonic acid ester according to claims 1 to 4, as a radiation-sensitive component in a radiation-sensitive mixture.

## Revendications

1. Procédé pour la préparation d'esters d'acides 1,2-naphtoquinone-(2)-diazide-4-sulfoniques de formule générale I 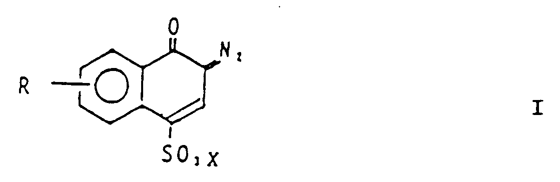 qui sont substitués en au moins l'une des positions 5, 6, 7 ou 8 par R = un atome d'halogène ou un groupe alcoxy ou alcoxycarbonyle, et dans lesquels X représente un groupe alkyle, par la combinaison d'étapes de processus connues en soi, dans lequel
a) on soumet à une nitrosation un β-naphtol convenablement substitué,
b) on le réduit et le soumet à une sulfonation en position 4 avec un hydrogénosulfite alcalin et un acide,
c) on oxyde le dérivé d'acide naphtalènesulfonique,
d) on fait réagir l'acide 1,2-naphtoquinone-4-sulfonique formé avec du toluènesulfonhydrazide dans un solvant organique, à des températures de 20 à 100°C,
e) on convertit en le chlorure de sulfonyle le composé naphtoquinonediazide résultant, à l'aide d'acide chlorosulfonique ou d'acide chlorosulfonique/chlorure de thionyle, et
f) on le condense ensuite avec un composant phénolique.

2. Procédé selon la revendication 1, caractérisé en ce que l'on utilise des β-naphtols qui sont substitués par l'atome de brome ou des groupes alcoxy ayant de 1 à 4 atomes de carbone ou des groupes alcoxycarbonyle ayant de 2 à 5 atomes de carbone.

3. Procédé selon la revendication 1 ou 2, caractérisé en ce que l'on utilise des β-naphtols qui sont substitués par des groupes méthoxy ou méthoxycarbonyle.

4. Procédé selon la revendication 3, caractérisé en ce que l'on utilise un β-naphtol qui est substitué par des groupes méthoxycarbonyle.

5. Procédé selon les revendications 1 à 4, caractérisé en ce que, pour la préparation du chlorure de sulfonyle, on utilise le mélange des isomères acide 1,2-naphtoquinone-(2)-diazide-4-sulfonique et acide 1,2-naphtoquinone-(1)-diazide-4-sulfonique, et on effectue la séparation des isomères par hydrolyse.

6. Procédé selon les revendications 1 à 5, caractérisé en ce que, pour la condensation avec des phénols, on utilise des bases à empêchement stérique, de préférence le 1,4-diazabicyclo[2.2.2]octane.

7. Procédé selon les revendications 1 à 6, caractérisé en ce que, en tant que composant phénolique, on utilise un dérivé d'hydroxybenzophénone ou une Novolaque.

8. Utilisation de l'ester d'acide 6-alcoxycarbonyl-1,2-naphtoquinone-(2)-diazide-4-sulfonyle selon les revendications 1 à 4, en tant que composant sensible aux radiations, dans une composition sensible aux radiations.
